# EUROPEAN PATENT APPLICATION

(11) **EP 4 524 231 A1**
(43) Date of publication of application: **19.03.2025**
(21) Application number: 23825991.5
(22) Date of filing: 06.05.2023
(51) Int. Cl.: C12M 3/00, C12M 1/38, C12M 1/36, C12M 1/34, C12M 1/26, C12M 1/24, C12M 1/12, C12M 1/04, C12M 1/00

(54) **CELL CULTURE METHOD, CELL CULTURE CONTAINER, AND CELL CULTURE DEVICE**

(30) Priority: 24.06.2022 CN 202210726988
(71) Applicant: Changsha Chusi Weikang Intelligent Technology Co., Ltd, Changsha, Hunan 410013 (CN)
(72) Inventor: HU, Biliang, Ningxiang Changsha, Hunan 410600 (CN); TAN, Liang, Ningxiang Changsha, Hunan 410600 (CN); JIANG, Hongwei, Ningxiang Changsha, Hunan 410600 (CN)
(74) Representative: Chung, Hoi Kan
(86) International application number: PCT/CN2023/092606
(87) International publication number: WO 2023/246341

(57) **Abstract**

Disclosed in the present invention is a cell culture method, including the following steps: first loading a one-step container body (1) into a microenvironment; adding a cell culture solution to the one-step container body (1); and successively adding a supplementary fluid to the one-step container body (1) as the culture time increases. The one-step container body (1) has at least two inner side surfaces with different areas. The inner side surfaces with the different areas form corresponding culture surfaces (2) for culturing cell solutions with different volumes. The cell solutions are first borne on a small-area culture surface (2) to be cultured. As the volumes of the cell solutions increase, the cell solution is borne on the large-area culture surface (2) to be cultured. Further disclosed is a cell culture container, including a one-step container body (1). The one-step container body (1) has at least two inner side surfaces having different areas. The inner side surfaces having the different areas form corresponding culture surfaces (2) for culturing cell solutions having different volumes. The cell culture method and the cell culture container can satisfy culture requirements of the cell solution, improve the cell culture effect, easily achieve automatic production, and have high production efficiency.

## Description

### TECHNICAL FIELD

The present invention relates to the technical field of food, medicine and life science, and in particular to a cell culture method, a cell culture container and a cell culture device.

### BACKGROUND

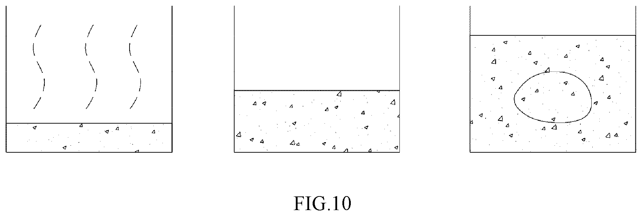

Cell culture refers broadly to all in vitro culture, which means that a tissue is removed from a living animal, placed under specific in vivo conditions simulating an in vivo physiological environment, and the like and incubated and cultured so as to survive and grow. Due to the advantage of a large gas exchange area, a commonly used cell culture bag is conducive to the culture of a cell so that more high-quality cells are obtained. Further, a variety of operations such as microscopic observation and monitoring of a cell culture process can be directly performed to ensure the entire process of asepsis and ease of operation. Therefore, the commonly used cell culture bag is widely used. As shown in FIG. 10, A less amount of cell solution is tiled as an initial amount of cell culture in a larger volume of a cell culture container, which causes faster evaporation of the cell solution, causes the osmotic pressure of a culture medium to be too high, causes cell crumpling, even causes cellular dysfunction, and ultimately leads to the apoptosis of the cells. A larger amount of the cell solution as the initial amount of the cell culture has a too low cell density and a slow expansion speed. In vitro, the logarithmic proliferation of the cells needs the interaction between the cells. When the density of the cells is lower, the proliferation rate of the cells is significantly reduced. In addition, a larger amount of the cell solution as the initial amount of the cell culture not only causes a waste of cost, but also requires more blood to be collected from a patient, which is also undesirable.

Chinese Patent Application No. 201822257139.0 discloses a novel cell culture bag. A sealing groove and a sealing strip are designed in the middle of the culture bag and accompanied with a drainage port on the culture bag so that a half of a culture solution in the culture bag can be quantitatively extracted.

Chinese Patent Application No. 201510906678.0 discloses a three-dimensional structure cell culture bag. This invention proposes a three-dimensional structure cell culture bag with a reagent connector, a sensor and a sampling bag that are integrated, which more effectively uses a closed culture space compared with a flat culture bag.

Chinese Patent Application No. 202020480642.7 discloses a variable-volume cell culture bag. This invention proposes a cell culture bag. With a separating clip on a body of the bag, the volume of the culture bag can be adjusted, to meet the culture demand in a less cell solution as the initial amount of cell culture, and at the same time can collect and monitor PH and DO in the course of the cell culture.

Chinese Patent Application No. 202110782370.5 discloses a large-capacity suspension cell culture flask. This invention improves a cell culture effect by installing a gas permeability membrane on the upper and lower portions of the flask to increase the amount of gas permeability during a culture process.

The above patent literatures propose a part of solutions in the process of cell culture, but have the following shortcomings: difficulty in meeting the culture demand of an increasing volume; poor cell culture effect; difficulty in realizing automatic production; and low production efficiency.

### SUMMARY

The technical problem to be solved by the present invention is to overcome the deficiencies in the prior art. Therefore, the present invention provides a cell culture method, a cell culture container, and a cell culture device, which are capable of meeting the culture demand of a cell solution having an ever-increasing volume, improving a cell culture effect, and easily realizing automatic production with high production efficiency.

To resolve the foregoing technical problem, the following technical solutions are used in the present invention:
A cell culture method includes the following steps: first loading a clean and sterile one-step container body into a microenvironment for cell culture; adding a cell culture solution required for the cell culture to the one-step container body; and successively adding a supplementary fluid required for the cell culture to the one-step container body as culture time increases, where the one-step container body has at least two inner side surfaces with different areas; the inner side surfaces with the different areas form corresponding culture surfaces for culturing cell solutions with different volumes; the cell solutions are first borne on a small-area culture surface to be cultured; as the volumes of the cell solutions increase, the one-step container body is rotated so that the cell solutions are borne on a large-area culture surface to be cultured, which then completes one time of continuous cell culture.

The cell culture solution includes a fluid required for the cell culture such as a cell fluid and a culture fluid. The supplementary fluid includes a nutrient used for the growth and reproduction of the cells such as a culture medium. The cell solution is also referred to as a cell suspension fluid.

As a further improvement of the above technical solution:
During the process of culture of the cell solution on the culture surface, the one-step container body is swayed so that the cell distribution and a temperature of the cell solution in the one-step container body are uniform, thereby increasing an exchange rate of a gas in the cell solution.

During the process of culture of the cell solution, the one-step container body is periodically rotated to a collection position. After the one-step container body stands for a specified length of time, real-time cell information of the cells is collected and monitored. After the cell solution reaches a desired concentration, the one-step container body is rotated to a supernatant discharge position and stands for a specified length of time, so that the cultured cells are fully precipitated. Then, a part of a supernatant in the one-step container body is discharged, so as to reduce a total volume of the cell solution.

As one general inventive concept, the present invention also provides a cell culture container, including an one-step container body. The one-step container body has at least two inner side surfaces with different areas. The inner side surfaces with the different areas form corresponding culture surfaces for culturing cell solutions with different volumes. The one-step container body is provided with a flow channel. The one-step container body is provided with a gas permeable membrane or a gas channel for gas exchange.

As a further improvement of the above technical solution:
The one-step container body is a rigid one-step culture flask. The one-step container body includes two end covers disposed opposite to each other. The end cover is provided with a rotating seat. The culture surfaces are disposed in parallel around a rotation axis of the one-step container body to form a polygonal perimeter edge.

The flow channel is disposed on a rotation axis of the end cover and extends to the perimeter edge. At least two flow channels are provided. One of the two flow channels is configured to discharge a supernatant.

The flow channel extends over the culture surface with the minimum area. An observation window for collecting an image and/or a sampling port for sampling is disposed on the one-step container body.

A plurality of gas permeability holes are disposed on a side wall where the culture surface is located. A layer of gas permeability membrane that is permeable to a gas and impermeable to a solution is laid on the culture surface.

The one-step container body is a flexible one-step culture bag. A shaping sleeve is disposed outside the one-step container body. The shaping sleeve is configured to fix the one-step container body to form the culture surfaces having the different areas.

A plurality of fixing portions are disposed in the shaping sleeve. The one-step container body is detachably fixed in the shaping sleeve by means of each of the fixing portions. An installing port is disposed at the side of the shaping sleeve. The shaping sleeve is provided with a sealing cover at the installing port. A rotating seat is disposed on the shaping sleeve.

A heating mechanism is disposed on the side wall of the shaping sleeve corresponding to each of the culture surfaces.

As one general inventive concept, the present invention further provides a cell culture device, including a culture chamber and a cell culture container. An one-step container body is detachably mounted in the culture chamber. A rotary drive mechanism for driving the rotation of the one-step container body is disposed on the culture chamber.

As a further improvement of the above technical solution:
A primary rotary connector and a secondary rotary connector are disposed on the two sides of the culture chamber, respectively. The one-step container body is detachably mounted between the primary rotary connector and the secondary rotary connector. The primary rotary connector is connected to the rotary drive mechanism.

Compared with the prior art, the present invention has the following advantages:
For the cell culture method of the present invention, as the volume of the cell solution increases, by rotating the one-step container body, the cell solution is borne on the culture surface with the corresponding area to be cultured. Therefore, the concentration of the cell solution is not too low, thereby preventing faster evaporation of the cell solution. The concentration of the cell solution is not too high, thereby preventing too low density of the cells and slow amplification, meeting the culture demand of an ever-increasing volume of the cell solution, and improving a cell culture effect. Moreover, the one-step container body is rotated and adjusted to different culture surfaces, which mainly realizes the cell culture method. Therefore, a one-step continuous cell culture is completed, which can adopt a single use container or a reusable container. Therefore, automatic production is conveniently carried out with an automatic system, which has high production efficiency, solves the problem of an existing troublesome culture operation, realizes automation, and reduces the amount and cost of the one-step container body.

For the cell culture container of the present invention, a cell culture process is as follows: as the volume of the cell solution increases, by rotating the one-step container body, the cell solution is borne to the culture surface with the corresponding area to be cultured. Therefore, the concentration of the cell solution is not too low, thereby preventing faster evaporation of the cell solution. The concentration of the cell solution is not too high, thereby preventing too low density of the cells and slow amplification, meeting the culture demand of an ever-increasing volume, and improving a cell culture effect. Moreover, the one-step container body is rotated and adjusted to different culture surfaces, which mainly realizes the cell culture container. Automatic production is carried out with the automatic system, which has high production efficiency.

For the cell culture container of the present invention, with an increase in the volume of the cell solution, only a small angle is rotated, so that the corresponding larger culture surface is positioned at the bottom and horizontally arranged to load the cell solution, which has high efficiency and low power consumption.

For the cell culture container of the present invention, a plurality of gas permeability holes and a gas permeability membrane on the side wall of the culture surface together constitute a gas exchange system of the cell culture container, thereby improving a gas exchange amount during a culture process.

For the cell culture container of the present invention, the shaping sleeve is a hard structure. The cell culture container is a body combined by softness and hardness, which is convenient to manufacture.

The cell culture container of the present invention, the one-step container body is detachably fixed in the shaping sleeve with each fixing portion, which facilitates the removal and replacement of the one-step container body. The shaping sleeve is provided with a sealing cover at the installing port. When the one-step container body is removed and replaced, the sealing cover can be opened. Then, the fixing of the one-step container body by the fixing portion is dismantled, so as to take out the one-step container body. A structure is simple and easy to operate.

The cell culture device of the present invention includes all technical features of the cell culture container and has all advantages of the cell culture container.

### BRIEF DESCRIPTION OF THE DRAWINGS

FIG. 1 is a schematic diagram of a three-dimensional structure of Embodiment I of a cell culture container of the present invention.
FIG. 2 is a side view schematic structural diagram of Embodiment I of a cell culture container of the present invention.
FIG. 3 is a schematic diagram of a sectional view structure at A-A in FIG. 2.
FIG. 4 is an enlarged view at B in FIG. 3.
FIG. 5 is a schematic diagram of a main view structure of Embodiment I of a cell culture container of the present invention.
FIG. 6 is a schematic diagram of a sectional view structure at C-C in FIG. 5.
FIG. 7 is a diagram of the use of different culture surfaces of Embodiment I of a cell culture container of the present invention.
FIG. 8 is an exploded view of Embodiment II of a cell culture container of the present invention.
FIG. 9 is a diagram of the use of different culture surfaces of Embodiment II of a cell culture container of the present invention.
FIG. 10 is a diagram of the relation of a cell solution and a cell culture container.
FIG. 11 is a schematic structural diagram of a cell culture device of the present invention.
FIG. 12 is a graph of an average number of cells for different days of culture of Embodiment II of a cell culture container of the present invention.
FIG. 13 is a curve graph of an average survival rate of cells for different days of culture for Embodiment II of a cell culture container of the present invention.
FIG. 14 is a curve graph of an average density of cells for different days of culture of Embodiment II of a cell culture container of the present invention.

Each of reference signs in the figures indicates:
1, one-step container body: 11, perimeter edge; 12, end cover; 121, flow channel; 13, flow channel: 14, gas channel; 2, culture surface; 21, gas permeability hole; 22, observation window; 23, sampling port; 24, virus filling port; 3, rotating seat; 4, gas permeability membrane; 5, shaping sleeve; 6, fixing portion; 7, installing port; 8, sealing cover; 9, heating mechanism: 91, incubation chamber; 92, rotary drive mechanism; 93, primary rotary connector; 94, secondary rotary connector.

### DETAILED DESCRIPTION OF THE EMBODIMENTS

The present invention will be further described below in detail in connection with the accompanying drawings of the specification and specific embodiments.

In the description of the present application, it should be understood that the orientation or positional relationship indicated by the terms such as "upper", "lower", "front", "rear", "horizontal", "bottom", "inner", "outer", etc. are based on the orientation or positional relationship shown in the drawings, and are only for the convenience of describing the present application and simplifying the description, rather than indicating or implying that a device or element referred to should have a specific orientation, be configured and operated in a specific orientation, and therefore cannot be understood as a limitation to the present application.

In the description of the present application, "a plurality of" refers to two or more, unless otherwise expressly and specifically defined.

In the present application, unless otherwise clearly specified and limited, terms such as "assemble", "connected", "connection", and "fixed" shall be understood in a general sense. For example, these technical terms may be a fixed connection, a detachable connection, or an integrated connection; or may be a mechanical connection or an electrical connection; or may be a direct connection, an indirect connection by using an intermediate medium, or an internal communication of two elements or an interaction of two elements. For a person skilled in the art, the specific meaning of the forgoing terms in the present application may be understood based on specific situations.

### Embodiment I:

A cell culture method of the embodiment included the steps: first loading a clean and sterile one-step container body 1 into a microenvironment for cell culture; adding a cell culture solution required for the cell culture to the one-step container body 1; and successively adding a supplementary fluid required for the cell culture to the one-step container body 1 as culture time increases. The one-step container body 1 had at least two inner side surfaces with different areas. The inner side surfaces with the different areas formed corresponding culture surfaces 2 for culturing cell solutions with different volumes. The cell solutions were first borne on a small-area culture surface 2 to be cultured. As the volumes of the cell solutions increased, the one-step container body 1 was rotated so that the cell solutions were borne on a large-area culture surface 2 to be cultured.

For the cell culture method of the embodiment, as the volumes of the cell solutions increased, by rotating the one-step container body 1, the cell solutions were borne to the culture surface 2 with the corresponding area to be cultured (in the premise of the same volume of the cell solution, the area of the culture surface 2 increased and the height of cell solution decreased). Therefore, the height n of the cell solution was not too low, thereby preventing faster evaporation of the cell solution. The height of the cell solution was not too high, thereby preventing too low density of the cells, slow amplification and the non-contact between the cells in the cell solution and air, meeting the culture demand of an ever-increasing volume of the cell solution, and improving a cell culture effect. Moreover, the one-step container body 1 was rotated and adjusted to different culture surfaces 2, which mainly realized the cell culture method. There was no need to change the culture container as the volume of the cell solution increases during the culture process. Therefore, a one-step continuous cell culture was completed, which could adopt a single use container or a reusable container. Therefore, automatic production was conveniently carried out with an automatic system, which had high production efficiency.

In this embodiment, during the process of culture of the cell solution on the culture surface 2, the one-step container body 1 was swayed so that the cell distribution and a temperature of the cell solution in the one-step container body 1 were uniform, thereby increasing the culture effect of the cells.

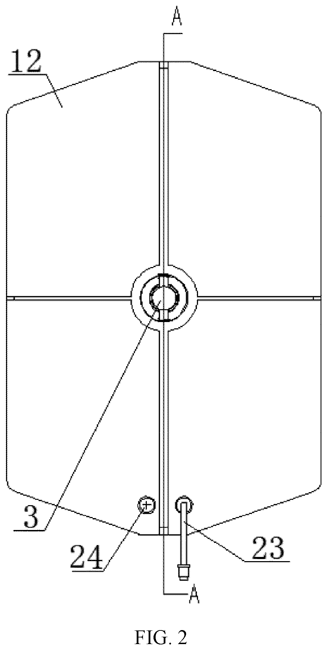

In this embodiment, during the process of culturing the cell solution on the culture surface 2, the one-step container body 1 was periodically rotated until the culture surface 2 with the smallest area was in a lower horizontal position. After the one-step container body stood for a specified length of time, real-time growth information of the cells was collected and monitored. The cell solution could also be extracted as a sample through the sampling port 23. After the cell solution reached the expected concentration, the one-step container body 1 was rotated until the culture surface 2 with the smallest area was in the lower horizontal position, at which time the one-step container body 1 was in a supernatant discharge position. After the one-step container body stood for a specified length of time, the cultured cells were fully precipitated. Then, a part of the supernatant in the one-step container body 1 was discharged, so as to reduce the total volume of cell solution. As shown in FIG. 2, a viral filling port 24 was disposed next to the sampling port 23 and configured to add lentivirus to carry out T-cell transfection in a pre-culture phase of the cell culture, so as to effect T-cells to stably express CAR.

### Embodiment II:

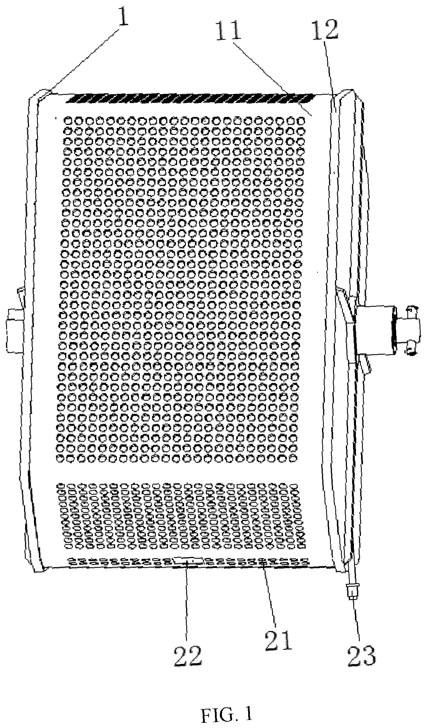

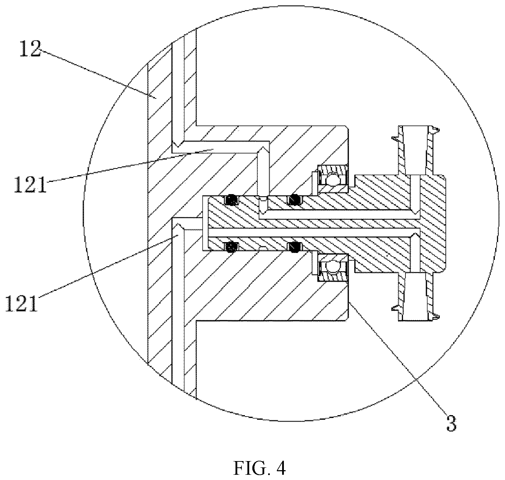

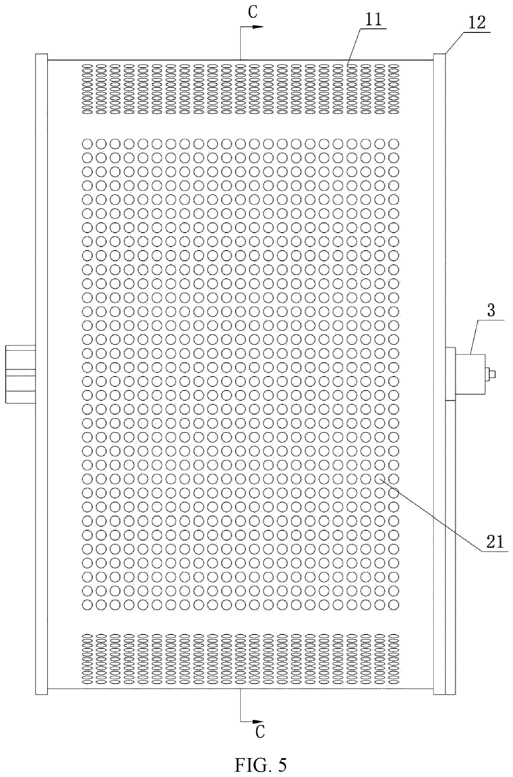

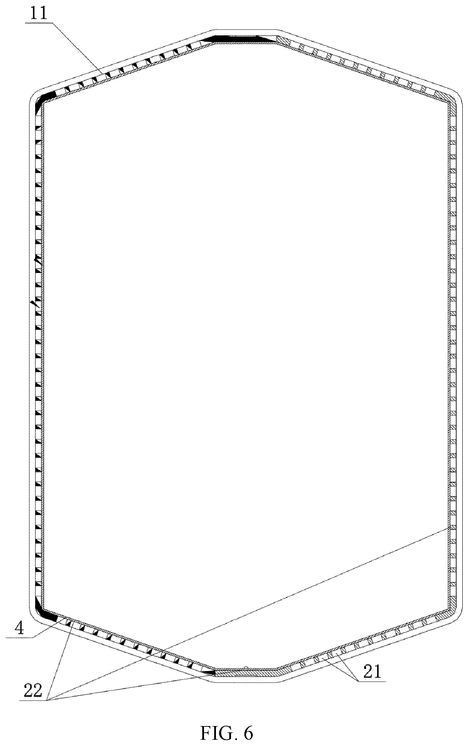

FIGS. 1-7 showed an embodiment of a cell culture container of the present invention, including an one-step container body 1. The one-step container body 1 had at least two inner side surfaces with different areas. The inner side surfaces with the different areas formed corresponding culture surfaces 2 for culturing cell solutions with different volumes.

For the cell culture process, as the volume of the cell solution increases, by rotating the one-step container body 1, the cell solution was borne on the culture surface 2 with the corresponding area to be cultured. Therefore, the height of the cell solution is not too low, thereby preventing faster evaporation of the cell solution. The height of the cell solution was not too high, thereby preventing too low density of the cells and slow amplification, meeting the culture demand of an ever-increasing volume of the cell solution, and improving a cell culture effect. Moreover, the one-step container body 1 was rotated and adjusted to different culture surfaces 2, which mainly realizes the cell culture container. Automatic production was carried out with the automatic system, which had high production efficiency.

In this embodiment, the one-step container body 1 was provided with a rotating seat 3. Each culture surface 2 was sequentially disposed around the rotation axis of the one-step container body 1. Each culture surface 2 was sequentially connected and the area of the culture surfaces was sequentially increased. Therefore, with an increase in the volume of the cell solution, only a small angle was rotated, so that the corresponding larger culture surface 2 was positioned at the bottom and horizontally arranged to load the cell solution, which had high efficiency and low power consumption.

In this embodiment, a plurality of gas permeability holes 21 were disposed on a side wall where the culture surface 2 is located. A layer of gas permeability membrane 4 that was permeable to a gas and impermeable to a solution was laid on the culture surface 2. A plurality of gas permeability holes 21 and the gas permeability membrane 4 on the side wall of the culture surface 2 together constituted a gas exchange system of the cell culture container, thereby improving a gas exchange amount during the culture process.

The one-step container body 1 is capable of swaying in the culture process, enhancing the cell distribution of the culture solution at different angle positions, the uniformity of the temperature and optimal gas permeability.

In this embodiment, the one-step container body 1 was a rigid one-step culture flask. The one-step container body 1 included a polygonal perimeter edge 11 and two end covers 12 disposed at the two ends of the perimeter edge 11, respectively. The culture surface 2 was formed on the inner side surface of the perimeter edge 11. The rotating seat 3 was disposed in the middle of the end cover 12. A flow channel 121 was communicated between the outer side of the rotating center of the end cover 12 and the inner side of the end cover near the narrowest culture surface 2. The flow channel 121 extended from the center of the end cover 12 to the perimeter edge 11. The rotating seat 3 and one end of the flow channel 121 were disposed in the center of the end cover 12. The flow channel 121 was disposed on the rotation axis to ensure that the position of the end of the flow channel did not change when rotating, thereby being convenient to replenish the supplementary fluid in the culture process. The side wall where the narrowest culture surface 2 was located was provided with an observation window 22 and a sampling port 23 for sampling.

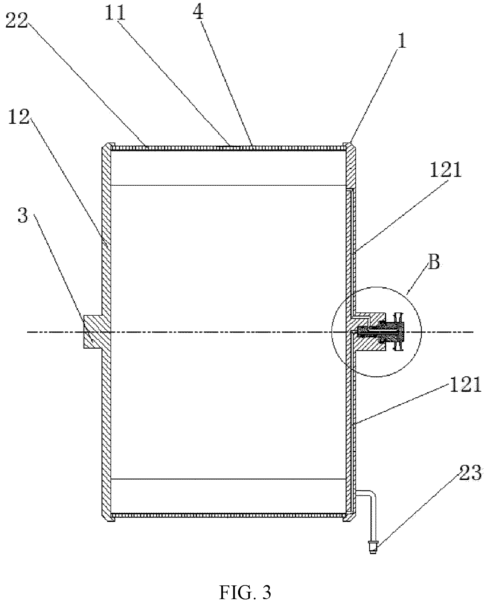

The two flow channels 121 were provided. One of the flow channels 121 was configured to discharge a supernatant. The supernatant came from the top of the cell solution. Therefore, the flow channel 121 extended to the top of the culture surface 2 and was at a certain distance from the culture surface 2. The other flow channel 121 needed to be used for the intake and outtake operations of the fluid. To ensure that all the solution could be completely discharged, the flow channel 121 needed to be extended to the culture surface 2. Further, for the cell culture that needed to be sampled, the flow channel 121 was sampled, which could result in waste in the cell solution because the flow channel 121 was too long. As shown in FIG. 3, the two flow channels 121 were arranged in a 180-degree according to the rotation axis. One of the flow channels 121 leaded to a small-area culture surface 2. An interface of the flow channel 121 is located at the small-area culture surface 2. The interface of the other of the flow channels 121 leaded to the small-area culture surface 2 at an opposite side. The interface at a certain height from the small-area culture surface 2 was configured to drain an upper part of the supernatant at the end of culture.

The one-step container body 1 is structured with the flow channel 121 and the rotating seat 3 to realize automatic addition of the supplementary fluid, automatic discharge of the supernatant, automatic harvesting, and the like, in the rotating process, thereby replacing manual operation.

During the process of culturing the cell solution on the culture surface 2, the one-step container body 1 was periodically rotated until the culture surface 2 with the smallest area was in a lower horizontal position. After the one-step container body stood for a specified length of time, real-time growth information of the cells was collected and monitored via the observation window 22. The collection position is a position where information could be collected from the observation window 22 by an information collection device such as a camera. In this embodiment, the observation window 22 was disposed on the culture surface 2 with the smallest area. After the cell solution reached the expected concentration, the one-step container body 1 was rotated until the culture surface with the smallest area 2 was in the lower horizontal position, at which time the one-step container body 1 was in a supernatant discharge position. After the one-step container body stood for a specified length of time, the cultured cells were fully precipitated. Then, a part of the supernatant in the one-step container body 1 was discharged via the flow channel 121, so as to reduce the total volume of cell solution.

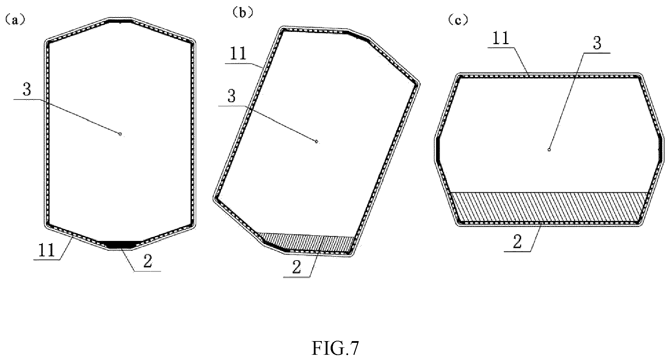

As shown in FIG. 7, in this embodiment, the culture surface 2 included a small surface, a medium surface, and a large surface whose culture area increased sequentially. FIG. 7(a) indicated that the cell solution was cultured using the small surface. FIG. 7(b) indicated that the cell solution was cultured using the medium surface. FIG. 7(c) indicated that the cell solution was cultured using the large surface. As the number of days of culture increased, the volume of the cell solution gradually increased. An angle of the one-step container body 1 was transformed to adjust the culture surface 2, i.e., for the culture, the small surface was first used; then the medium surface was used, and finally the large surface was used. The one-step container body 1 was swayed while culturing the cells.

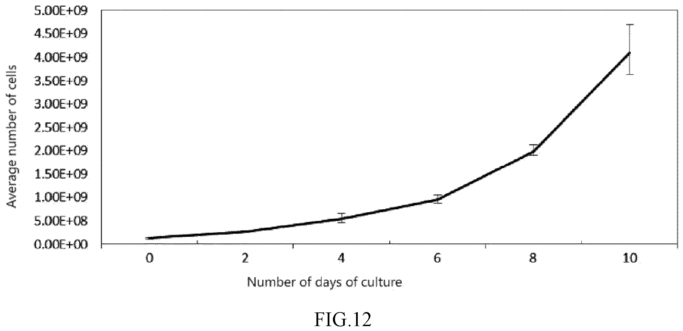

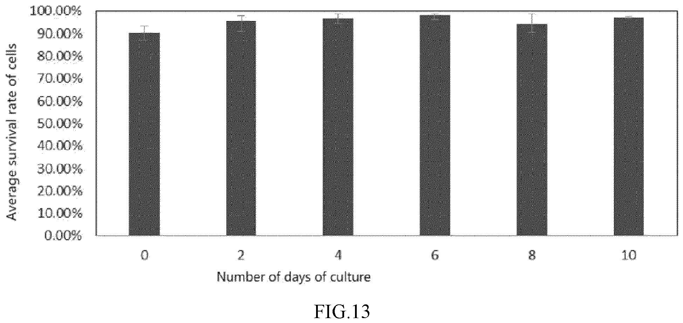

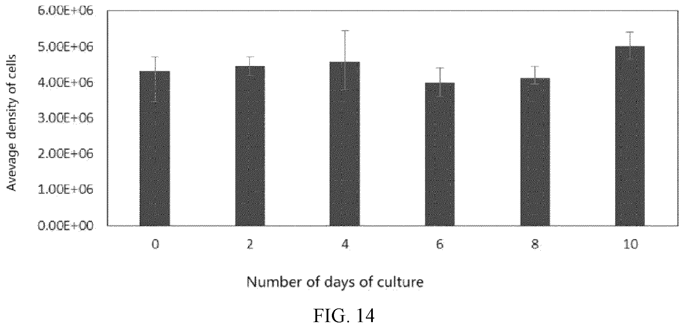

Tables I, II and III showed the cell numbers, survival rates and densities of different culture surfaces at different days of the culture, respectively. The first group, the second group, the third group and the fourth group denote four identical one-step container bodies 1, respectively. As can be seen from Table I, Table II, Table III, and FIGS. 12, 13, and 14, as the culture volume increased with an increase in the number of days of the culture. The culture surface 2 was transformed, which can prevent the increase in volume and the accumulation of the cell solution to the extent that the intermediate cells have a low rate of dissolved oxygen, and meets the culture demand for the cell solution with an ever-increasing volume to improve the effect of the cell culture.

**Table I Number of cells in different culture surfaces 2 at different days of culture**

| Culture surface | Culture volume | Number of days of culture | The first group | The second group | The third group | The fourth group | Average number |
|---|---|---|---|---|---|---|---|
| Small surface+ swaying | 30 | 0 | 1.04E+08 | 1.40E+08 | 1.34E+08 | 1.41E+08 | 1.29E+08 |
| Small surface+ swaying | 60 | 2 | 2.79E+08 | 2.52E+08 | 2.58E+08 | 2.82E+08 | 2.68E+08 |
| Medium surface +swaying | 120 | 4 | 5.58E+08 | 5.28E+08 | 4.56E+08 | 6.54E+08 | 5.49E+08 |
| Medium surface +swaying | 240 | 6 | 8.74E+08 | 8.64E+08 | 1.06E+09 | 1.04E+09 | 9.58E+08 |
| Large surface+ swaying | 480 | 8 | 1.92E+09 | 1.90E+09 | 1.97E+09 | 2.14E+09 | 1.98E+09 |
| Large surface+ swaying | 960 | 10 | 3.78E+09 | 3.63E+09 | 4.70E+09 | 4.24E+09 | 4.09E+09 |

**Table II Cell survival rates of different culture surfaces 2 at different days of culture**

| Culture surface | Culture volume | Number of days of culture | The first group | The second group | The third group | The fourth group | Average number |
|---|---|---|---|---|---|---|---|
| Small surface+ swaying | 30 | 0 | 91.30% | 87.10% | 93.26% | 89.36% | 90.26% |
| Small surface+ swaying | 60 | 2 | 97.89% | 90.96% | 96.23% | 97.37% | 95.61% |
| Medium surface +swaying | 120 | 4 | 94.62% | 95.45% | 98.68% | 98.17% | 96.73% |
| Medium surface +swaying | 240 | 6 | 98.90% | 98.89% | 98.18% | 96.30% | 98.07% |
| Large surface+ swaying | 480 | 8 | 90.48% | 95.06% | 98.78% | 92.75% | 94.27% |
| Large surface+ swaying | 960 | 10 | 96.99% | 97.78% | 96.55% | 97.44% | 97.19% |

**Table III Cell densities of different culture surfaces 2 at different days of culture**

| Culture surface | Culture volume | Number of days of culture | The first group | The second group | The third group | The fourth group | Average number |
|---|---|---|---|---|---|---|---|
| Small surface+ swaying | 30 | 0 | 3.45E+06 | 4.65E+06 | 4.45E+06 | 4.70E+06 | 4.31E+06 |
| Small surface+ swaying | 60 | 2 | 4.65E+06 | 4.20E+06 | 4.30E+06 | 4.70E+06 | 4.46E+06 |
| Medium surface +swaying | 120 | 4 | 4.65E+06 | 4.40E+06 | 3.80E+06 | 5.45E+06 | 4.58E+06 |
| Medium surface +swaying | 240 | 6 | 3.64E+06 | 3.60E+06 | 4.40E+06 | 4.32E+06 | 3.99E+06 |
| Large surface+ swaying | 480 | 8 | 4.00E+06 | 3.95E+06 | 4.10E+06 | 4.45E+06 | 4.13E+06 |
| Large surface+ swaying | 960 | 10 | 5.32E+06 | 5.40E+06 | 4.64E+06 | 4.68E+06 | 5.01E+06 |

### Embodiment III:

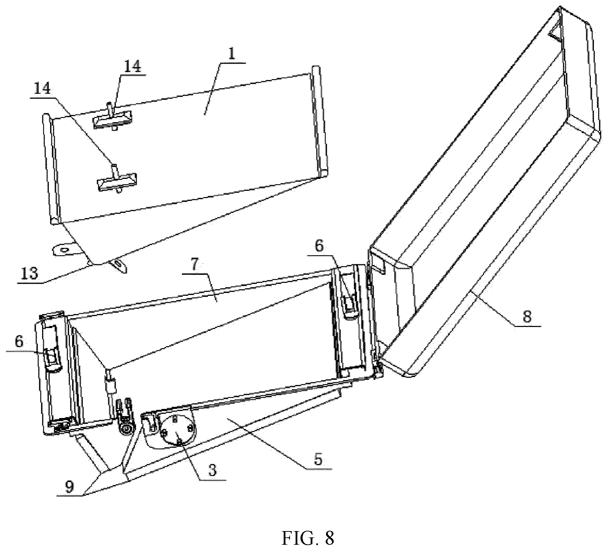

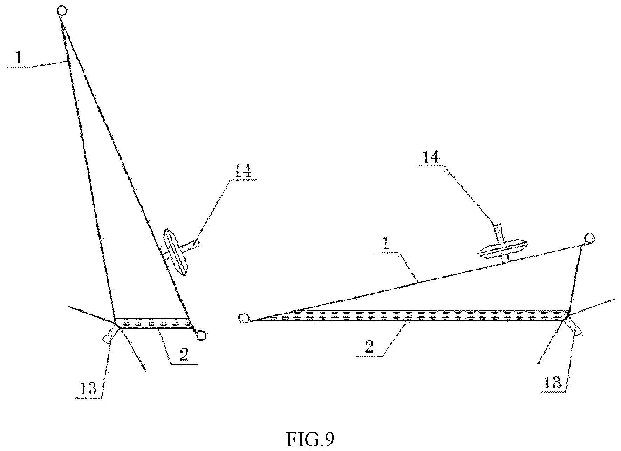

FIGS. 8 and 9 showed another embodiment of a cell culture container of the present invention, including an one-step container body 1. The one-step container body 1 had at least two inner side surfaces with different areas. The inner side surfaces with the different areas formed corresponding culture surfaces 2 for culturing cell solutions with different volumes. A shaping sleeve 5 was sleeved outside the one-step container body 1. The one-step container body 1 was a flexible one-step culture bag. The one-step culture bag was shaped by the shaping sleeve 5 to achieve the effect of having at least two inner surfaces with different areas, including but not limited to two, three, four or five, with the culture surfaces 2 being connected in sequence and the area increasing in sequence. A rotating seat 3 was disposed on the shaping sleeve 5. Each culture surface 2 was sequentially disposed around a center of rotation of the one-step container body 1. The shaping sleeve 5 was a hard structure. The cell culture container was a body combined by softness and hardness, which was convenient to manufacture. In other embodiments, the shaping sleeve 5 was prepared from metal or non-metallic material such as plastic and rubber. The shaping sleeve 5 was used in conjunction with the one-step container body 1. In some embodiments, the culture volume could be tens of milliliters to thousands of milliliters, or liters to thousands of liters.

A flow channels 13 and a gas channel 14 for gas exchange were disposed on the one-step container body 1. The two flow channels 13 were provided. One was for sampling, and the other was for entering or exiting a fluid. The two gas channels 14 were also provided. One was for gas inlet, and the other was for gas outlet.

In this embodiment, a plurality of fixing portions 6 were disposed in the shaping sleeve 5. The one-step container body 1 was detachably fixed in the shaping sleeve 5 by means of each of the fixing portions 6. An installing port 7 was disposed at the side of the shaping sleeve 5. The shaping sleeve 5 was provided with a sealing cover 8 at the installing port 7. The one-step container body 1 was detachably fixed in the shaping sleeve 5 with each fixing portion 6, which facilitates the removal and replacement of the one-step container body 1. The shaping sleeve 5 was provided with the sealing cover 8 at the installing port 7. When the one-step container body 1 was removed and replaced, the sealing cover 8 could be opened. Then, the fixing of the one-step container body 1 by the fixing portion 6 was dismantled, so as to take out the one-step container body 1. A structure is simple and easy to operate.

In this embodiment, a heating mechanism 9 was disposed on side walls corresponding to the shaping sleeve 5 and each of the culture surfaces 2. It is convenient to heat the cell solution.

### Embodiment IV:

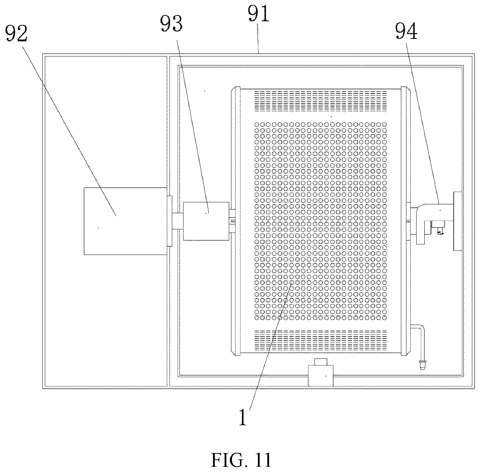

FIG. 11 showed an embodiment of a cell culture device of the present invention. The cell culture device of this embodiment included a culture chamber 91 and a cell culture container of Embodiment I. An one-step container body 1 was detachably mounted in the culture chamber 91. A rotary drive mechanism 92 for driving the rotation of the one-step container body 1 was disposed on the culture chamber 91.

The one-step container body 1 was rotated by the driving action of the rotary drive mechanism 92, so that the cell solution was cultured on a culture surface 2 with a non-corresponding area. The cell culture device included all technical features of the cell culture container and had all advantages of the cell culture container.

In this embodiment, a primary rotary connector 93 and a secondary rotary connector 94 were disposed on the two sides of the culture chamber 91, respectively. The one-step container body 1 was detachably mounted between the primary rotary connector 93 and the secondary rotary connector 94. The primary rotary connector 93 was connected to the rotary drive mechanism 92. The one-step container body 1 was detachably mounted between the primary rotary connector 93 and the secondary rotary connector 94. For example, the two sides of the one-step container body 1 were snap-fastened to the primary rotary connection head 93 and the secondary rotary connection head 94 for easy disassembly and assembly, respectively. The rotary drive mechanism 92 was configured to drive the primary rotary connector 93 to rotate, thereby driving the one-step container body 1 to rotate. Inlet and outlet door bodies were disposed on the side of a culture chamber 91 for entering and exiting the one-step container body 1.

Although the present invention has been disclosed as above by way of a preferable embodiment, the embodiment is however not intended to limit the present invention. A person skilled in the art, without departing from the scope of the technical solution of the present invention, can make many possible changes and modifications to the technical solution of the present invention by utilizing the technical contents disclosed above, or modify the equivalent embodiments into equivalent changes. Therefore, any simple amendments, equivalent changes and modifications made to the above embodiments based on the technical substance of the present invention without departing from the content of the technical scheme of the present invention shall fall within the scope of protection of the technical solution of the present invention.

## Claims

1. A cell culture method, comprising the following steps: first loading a clean and sterile one-step container body (1) into a microenvironment for cell culture; adding a cell culture solution required for the cell culture to the one-step container body (1); and successively adding a supplementary fluid required for the cell culture to the one-step container body (1) as culture time increases, wherein the one-step container body (1) has at least two inner side surfaces with different areas, the inner side surfaces with the different areas form corresponding culture surfaces (2) for culturing cell solutions with different volumes, the cell solution is first borne on a small-area culture surface (2) to be cultured, as the volume of the cell solution increases, the one-step container body (1) is rotated so that the cell solution is borne on a large-area culture surface (2) to be cultured, and each of the culture surfaces (2) is disposed around a rotation axis of the one-step container body (1).

2. The cell culture method according to claim 1, wherein during a process of culture of the cell solution on the culture surface (2), the one-step container body (1) is swayed so that cell distribution and a temperature of the cell solution in the one-step container body (1) are uniform.

3. The cell culture method according to claim 1, wherein during the process of the culture of the cell solution, the one-step container body (1) is periodically rotated to a collection position, after the one-step container body stands for a specified length of time, real-time cell information of cells is collected and monitored; after the cell solution reaches a desired concentration, the one-step container body (1) is rotated to a supernatant discharge position and stands for a specified length of time, so that the cultured cells are fully precipitated, then, a portion of a supernatant in the one-step container body (1) is discharged, so as to reduce total volumes of the cell solutions.

4. A cell culture container, comprising an one-step container body (1), wherein the one-step container body (1) has at least two inner side surfaces with different areas, the inner side surfaces with the different areas form corresponding culture surfaces (2) for culturing cell solutions with different volumes, the one-step container body (1) is provided with a flow channel (121, 13), the one-step container body (1) is provided with a gas permeability membrane (4) or a gas channel (14) for gas exchange, and each of the culture surfaces (2) is disposed around a rotation axis of the one-step container body (1).

5. The cell culture container according to claim 4, wherein the one-step container body (1) is a rigid one-step culture flask, the one-step container body (1) comprises two end covers (12) disposed opposite to each other, the end cover (12) is provided with a rotating seat (3), the culture surfaces (2) are disposed in parallel around a rotation axis of the one-step container body (1) to form a polygonal perimeter edge (11).

6. The cell culture container according to claim 5, wherein the flow channel (121) is disposed on a rotation axis of the end cover (12) and extends to the perimeter edge (11), at least two flow channels (121) are provided, and one of the two flow channels (121) is configured to discharge a supernatant.

7. The cell culture container according to claim 6, wherein the flow channel (121) extends over the culture surface (2) with a minimum area, an observation window (22) for collecting an image and/or a sampling port (23) for sampling is disposed on the one-step container body (1).

8. The cell culture container according to claim 4, wherein the one-step container body (1) is a flexible one-step culture bag, a shaping sleeve (5) is disposed outside the one-step container body (1), the shaping sleeve (5) is configured to fix the one-step container body (1) to form the culture surfaces (2) with the different areas.

9. The cell culture container according to claim 8, wherein a plurality of fixing portions (6) are disposed in the shaping sleeve (5), the one-step container body (1) is detachably fixed in the shaping sleeve (5) by means of each of the fixing portions (6), an installing port (7) is disposed at a side of the shaping sleeve (5), the shaping sleeve (5) is provided with a sealing cover (8) at the installing port (7), and a rotating seat (3) is disposed on the shaping sleeve (5).

10. The cell culture container according to claim 8 or 9, wherein a heating mechanism (9) is disposed on the shaping sleeve (5).

11. A cell culture device, comprising a culture chamber (91) and the cell culture container according to claims 4-9, a one-step container body (1) is detachably mounted in the culture chamber (91), a rotary drive mechanism (92) for driving rotation of the one-step container body (1) is disposed on the culture chamber (91).

12. The cell culture device according to claim 11, wherein a primary rotary connector (93) and a secondary rotary connector (94) are disposed on two sides of the culture chamber (91), respectively, the one-step container body (1) is detachably mounted between the primary rotary connector (93) and the secondary rotary connector (94), and the primary rotary connector (93) is connected to the rotary drive mechanism (92).

## Amended claims

### Amended claims under Art. 19.1 PCT

1. A cell culture method, comprising the following steps: first loading a clean and sterile one-step container body (1) into a microenvironment for cell culture; adding a cell culture solution required for the cell culture to the one-step container body (1); and successively adding a supplementary fluid required for the cell culture to the one-step container body (1) as culture time increases, wherein the one-step container body (1) has at least two inner side surfaces with different areas, the inner side surfaces with the different areas form corresponding culture surfaces (2) for culturing cell solutions with different volumes, the cell solution is first borne on a small-area culture surface (2) to be cultured, as the volume of the cell solution increases, the one-step container body (1) is rotated so that the cell solution is borne on a large-area culture surface (2) to be cultured, and each of the culture surfaces (2) is disposed around a rotation axis of the one-step container body (1).

2. The cell culture method according to claim 1, wherein during a process of culture of the cell solution on the culture surface (2), the one-step container body (1) is swayed so that cell distribution and a temperature of the cell solution in the one-step container body (1) are uniform.

3. The cell culture method according to claim 1, wherein during the process of the culture of the cell solution, the one-step container body (1) is periodically rotated to a collection position, after the one-step container body stands for a specified length of time, real-time cell information of cells is collected and monitored; after the cell solution reaches a desired concentration, the one-step container body (1) is rotated to a supernatant discharge position and stands for a specified length of time, so that the cultured cells are fully precipitated, then, a portion of a supernatant in the one-step container body (1) is discharged, so as to reduce total volumes of the cell solutions.

4. A cell culture container, comprising an one-step container body (1), wherein the one-step container body (1) has at least two inner side surfaces with different areas, the inner side surfaces with the different areas form corresponding culture surfaces (2) for culturing cell solutions with different volumes, the one-step container body (1) is provided with a flow channel (121, 13), the one-step container body (1) is provided with a gas permeability membrane (4) or a gas channel (14) for gas exchange, and each of the culture surfaces (2) is disposed around a rotation axis of the one-step container body (1).

5. The cell culture container according to claim 4, wherein the one-step container body (1) is a rigid one-step culture flask, the one-step container body (1) comprises two end covers (12) disposed opposite to each other, the end cover (12) is provided with a rotating seat (3), the culture surfaces (2) are disposed in parallel around a rotation axis of the one-step container body (1) to form a polygonal perimeter edge (11).

6. The cell culture container according to claim 5, wherein the flow channel (121) is disposed on a rotation axis of the end cover (12) and extends to the perimeter edge (11), at least two flow channels (121) are provided, and one of the two flow channels (121) is configured to discharge a supernatant.

7. The cell culture container according to claim 6, wherein the flow channel (121) extends over the culture surface (2) with a minimum area, an observation window (22) for collecting an image and/or a sampling port (23) for sampling is disposed on the one-step container body (1).

8. The cell culture container according to claim 4, wherein the one-step container body (1) is a flexible one-step culture bag, a shaping sleeve (5) is disposed outside the one-step container body (1), the shaping sleeve (5) is configured to fix the one-step container body (1) to form the culture surfaces (2) with the different areas.

9. The cell culture container according to claim 8, wherein a plurality of fixing portions (6) are disposed in the shaping sleeve (5), the one-step container body (1) is detachably fixed in the shaping sleeve (5) by means of each of the fixing portions (6), an installing port (7) is disposed at a side of the shaping sleeve (5), the shaping sleeve (5) is provided with a sealing cover (8) at the installing port (7), and a rotating seat (3) is disposed on the shaping sleeve (5).

10. The cell culture container according to claim 8 or 9, wherein a heating mechanism (9) is disposed on the shaping sleeve (5).

11. A cell culture device, comprising a culture chamber (91) and the cell culture container according to claims 4-9, a one-step container body (1) is detachably mounted in the culture chamber (91), a rotary drive mechanism (92) for driving rotation of the one-step container body (1) is disposed on the culture chamber (91).

12. The cell culture device according to claim 11, wherein a primary rotary connector (93) and a secondary rotary connector (94) are disposed on two sides of the culture chamber (91), respectively, the one-step container body (1) is detachably mounted between the primary rotary connector (93) and the secondary rotary connector (94), and the primary rotary connector (93) is connected to the rotary drive mechanism (92).
